Europäisches Patentamt

European Patent Office

Office européen des brevets

(11) Publication number: **0 658 565 A1**

## (12) EUROPEAN PATENT APPLICATION

(21) Application number: **93310259.2**

(22) Date of filing: **17.12.93**

(51) Int. Cl.6: **C07H 11/04**, A61K 7/16, A61K 7/06, A61K 7/32, A61K 7/48

(43) Date of publication of application:
**21.06.95 Bulletin 95/25**

(84) Designated Contracting States:
**DE FR GB IT**

(71) Applicant: **UNILEVER PLC**
**Unilever House**
**Blackfriars**
**London EC4P 4BO (GB)**

(72) Inventor: **Traudt, Michael David, c/o Unilever**
**Research Port**
**Sunlight Laboratory, Ouarry Road East,**
**Bebington**
**Wirral, Merseyside L633JW (GB)**
Inventor: **Waterfield, Philip Christopher, c/o**
**Unilever**
**Research Port Sunlight Laboratory,Ouarry**
**Road East**
**Bebington Wirral, Merseyside L633JW (GB)**

(74) Representative: **van Gent, Jan Paulus et al**
**Unilever N.V.,**
**Patent Division**
**Postbus 137**
**NL-3130 AC Vlaardingen (NL)**

(54) **Oral products.**

(57) The present invention relates to copper, stannous and zinc salts of polyhydroxy compounds having at least one acid, ester-linked salt-forming substituent. These salts have anti-bacterial activity and are useful for inclusion in personal care compositions, particularly in oral care compositions, to impart anti-plaque, anti-caries, anti-gingivitis properties thereto. Several of these salts are novel compounds; typical examples are stannous and copper salts of glucose-6-phosphate.

EP 0 658 565 A1

The present invention relates to copper, stannous and zinc salts of polyhydroxy compounds having at least one acid, ester-linked salt-forming substituent, having utility as therapeutically active ingredient in personal care compositions, particularly oral care compositions.

More particularly, the present invention related to copper, stannous and zinc salts of polyhydroxy compounds having at least 4 C-atoms, having at least one acid, ester linked salt-forming substituent, particularly saccharides having at least 4 carbon atoms, and to their utility as therapeutically active ingredients in oral care products.

Copper salts with a great variety of anions are known in the art as anti-plaque agents. Thus, US Patent 4,332,791 (Raaf et al) discloses quite a number of copper salts, including copper salts of organic acids such as copper citrate, copper lactate, copper gluconate and copper glycerophosphate.

Similarly, stannous salts with a great variety of anions have been described as anti-caries or anti-plaque agents in oral care products. Thus, e.g. in our EP 0,426,213 we have described many stannous salts with organic anions such as stannous gluconate, stannous citrate, stannous tartrate, having utility as anti-plaque agents in oral care products.

In EP 0,311,259, stannous gluconate in combination with stannous fluoride is disclosed for anti-staining anti-plaque and anti-gingivitis benefits.

Equally, zinc salts with a great variety of anions are well-known in the art as anti-plaque agents in oral care products. Thus, our above EP 0,426,213 also discloses a variety of zinc salts of organic acids such as zinc citrate, zinc glycerophosphate, zinc lactate, zinc tartrate as anti-plaque agents.

While it is primarily the copper-, stannous- or zinc cation which is responsible for the therapeutic activity of the copper-, stannous- or zinc compound, the anion nevertheless may also play an important role as regards stability, solubility, substantivity and overall efficacy of the compound.

We have now surprisingly found that copper-, stannous- or zinc salts of polyhydroxy compounds having at least 4 carbon-atoms and having at least one acid, ester-linked salt-forming substituent provide for improved anti-caries, anti-plaque and anti-gingivitis effects as compared with other copper-, stannous- or zinc salt. Furthermore, they are useful as anti-microbial agents in a variety of other products for personal care, such as deodorant-, anti-perspirant-, skin care-, hair care- and cosmetic compositions.

Several of the compounds of the invention are novel compounds, such as the copper-, stannous- and zinc salts of saccharides having at least 4 carbon-atoms and at least one acid, ester-linked salt-forming substituent, excluding, however, zinc hexosephosphates and stannous glucose-1-phosphate.

The present invention, therefore, relates to the above novel copper-, stannous or zinc compounds, as well as to their use and the use of zinc hexosephosphates and stannous glucose-1-phosphate as therapeutically active ingredients in oral care products and other personal care compositions.

The invention will now be further discussed in detail with particular reference to oral care compositions, it being understood that the invention is not limited thereto, other personal care compositions also being within the ambit of the present invention.

The polyhydroxy compound having at least one acid, ester-linked salt-forming substituent can be derived from any of the following materials: xylitol, lactitol, sorbitol, pentaerythritol, maltitol, monosaccharides such as galactose, glucose, fructose, arabinose, mannose, disaccharides such as lactose, maltose, melibiose, trisaccharides such as melezitose and raffinose etc..

Preferred materials are the monosaccharides.

The acid, ester-linked salt-forming substituent can be a phosphoric or polyphosphoric acid including pyrophosphoric acid residue, a carboxy residue such as a lactic acid-, citric- tartaric- or pyruvic acid residue, a boric acid residue, a sulphuric acid residue or a nitric acid residue. The preferred substituent is a phosphoric acid residue.

In the polyhydroxy molecule there is present at least one acid, ester-linked salt-forming substituent, and there may be up to 5 of such substituents.

In this respect it is observed that water-soluble salts of glycerophosphate, fructose-6-phosphate, sorbitol-6-phosphate, glucose-1-phosphate and glucose-6-phosphate have been proposed for inclusion in dental care products with remineralising properties (GB 1,222,197 Blendax).

Furthermore, in GB 1,009,957 and 1,115,370 (Colonial Sugar Refining Comp. Ltd.), calcium sugar phosphates are disclosed as anti-caries agents.

The compounds of the invention are copper, stannous and zinc salts. Of these, the stannous salts are preferred.

Naturally, the various salts can be mixed together, either with the same cation and different anions, or vice-versa.

The salts of the invention can be prepared in any suitable manner, e.g. by (poly)phosphorylating the appropriate polyhydroxy compound with (poly)phosphoric acid or phosphorus pentoxide or phosphorusoxytrichloride.

The copper-, stannous- or zinc salts of the present invention have particular utility as anti-bacterial, anti-plaque, anti-gingivitis, anti-bad breath or anti-caries agents in oral care products.

They may be used in such products in amounts ranging from 0.1-10 % by weight, usually 0.15-7.5 % by weight and preferably 0.2-5 % by weight of the oral care product. The oral care products can be in the form of toothpastes, gels, mouthwashes, powders, gargles, solutions, lozenges, chewing gum and the like.

The oral composition may furthermore comprise conventional ingredients, such as pharmaceutically acceptable carriers like starch, sucrose, polyols, surfactants, water or water/alcohol systems etc. When formulated into a dentifrice, such formulation may contain all the usual dentifrice ingredients. Thus, they may comprise particulate abrasive materials such as silicas, aluminas, calcium carbonates, dicalciumphosphates, hydroxyapatites, calcium pyrophosphates, trimetaphosphates, insoluble hexametaphosphates and so on, usually in amounts between 5 and 60% by weight.

Furthermore, the dentifrice formulations may comprise humectants such as glycerol, sorbitol, propyleneglycol, lactitol and so on.

Surface-active agents may also be included such as anionic, nonionic, amphoteric and zwitterionic synthetic detergents. Examples thereof are sodiumlaurylsulphate, sodium dodecylbenzenesulphonate, sodium mono- and dioctylphosphate, sodiumlauroylsarcosinate, cocamidopropylbetain

Binders and thickeners such as sodium carboxymethylcellulose, xanthan gum, gum arabic etc. may also be included, as well as synthetic polymers such as polyacrylates and carboxyvinyl polymers such as Carbopol®.

Flavours such as peppermint and spearmint oils may also be included, as well as preservatives, opacifying agents, colouring agents, pH-adjusting agents, sweetening agents and so on.

Addional anti-bacterial agents may also be included such as Triclosan, chlorhexidine, copper-, zinc- and stannous salts other than those according to the present invention, such as copper sulphate, zinc citrate and stannous pyrophosphate, sanguinarine extract, metronidazole. Further examples of additional anti-bacterial agents are quaternary ammonium compounds such as cetylpyridinium chloride; bisguanides such as chlorhexidine digluconate, hexetidine, octenidine, alexidine; halogenated bisphenolic compounds such as 2,2' methylenebis-(4-chloro-6-bromophenol).

Polymeric compounds which can enhance the delivery of active ingredients such as the anti-bacterial agents can also be included. Examples of such polymers are copolymers of polyvinylmethylether with maleic anhydride and other similar delivery enhancing polymers, e.g. those described in DE-A-3,942,643 (Colgate)

Furthermore anti-inflammatory agents such as ibuprofen, flurbiprofen, aspirin, indomethacin etc. may also be included.

Anti-caries agents such as sodium- and stannous fluoride, aminefluorides, sodium monofluorophosphate, calcium lactate and/or calcium glycerophosphates, strontium salts and strontium polyacrylates, casein and casein digests and phosphoproteins may also be included.

Other optional ingredients include vitamins such as Vitamin C, plant extracts, potassium salts such as potassium citrate, potassium chloride and potassium nitrate.

Other optional ingredients include enzymes such as dextranase and/or mutanase, amyloglucosidase, glucose-oxidase with lactoperoxidase, neuraminidases, and hydrogenperoxide generating compounds such as potassiumperoxydiphosphate.

Furthermore, the oral compositions may comprise anti-calculus agents such as alkalimetal pyrophosphates, hypophosphite-containing polymers, organic phosphonates, phosphocitrates etc..

Other optional ingredients that may be included are e.g. bacteriocins, bacteriophages, tissue respiratory factors, antibodies, bleaching agents such as peroxy compounds, effervescing systems such as sodium bicarbonate/citric acid systems, colour change systems, and so on.

The present invention will be further illustrated by way of Example.

## EXAMPLE 1

1 g (=3.3 m.mole) of disodium glucose 6-phosphate hydrate, obtained from Sigma Chemical Co., having a purity of 98-100 % based on max. 2 moles of $H_2O$ per molecule, was dissolved in 40 ml of cold de-ionised water. To this solution was added 0.62 g (=3.3 m.mole) of anhydrous stannous chloride, approx. 99 % pure, ex Sigma Chemical Co., and the resulting mixture was stirred for approx. 30 min. to allow complete reaction.

Subsequently, de-oxygenated methanol was added drop-wise to the mixture to cause the product to precipitate, and this was continued until the precipitation was complete. The precipitate was filtered rapidly

EP 0 658 565 A1

under a nitrogen atmosphere, washed thoroughly with excess methanol and dried in vacuo. The yield of stannous glucose-6-phosphate monohydrate was more than 90 %, which is typically almost quantitative.

All the above procedures were run under a strict nitrogen atmosphere, and the solutions were purged with nitrogen gas prior to their use.

The stannous glucose-6-phosphate (Sn G-6-P) monohydrate was further characterised as follows:

**Chemical Characterisation**

1. Physical data:

- white, odourless, crystalline powder at room temperature;
- decomposes at 148 °C to form a brown "oil"; and
- freely soluble in water, insoluble in methanol.

2. Infrared spectra:

| Peak Location (cm-1) | Type of Chemical Bond (stretch) |
|---|---|
| 3289 | -OH |
| 1636 | -OH |
| 1190 | $P = O$ |
| 1100-990 | P-O |
| 800-700 | C-C, C-H |
| 500-550 | Sn-O |

This spectrum supports the view that the product is a stannous phosphate salt. This differs from an organo-tin compound, where the Sn-ion is bound covalently to a carbon atom. Although the product features an organic ligand, the salt is actually an inorganic stannous compound by nature.

3. NMR spectra, in $D_2O$ at room temperature/270MHz:

a. proton (H1);

| Peak Location (ppm) | Number of Protons (H+) |
|---|---|
| 3.26 | 1 |
| 3.31 | 2 |
| 3.68 | 1 |
| 3.94 | 1 |
| 4.64 | 1 |
| 5.21 | 1 |

Proton NMR does not generally detect the H+ ions associated with -OH groups, so one is detecting the carbon-associated protons here. We have detected 7 H+ ions, which matches that expected for glucose 6-phosphate. Therefore, the Sn ion is not bound to a carbon.

4

EP 0 658 565 A1

b. Carbon-13;

| Peak Location (ppm) | Number of Carbons (C-13) |
|---|---|
| 61.6 | 1 (C6) |
| 66.4 | 1 (C3) |
| 67.6 | 1 (C4) |
| 69.8 | 1 (C2) |
| 89.3 | 1 (C5) |
| 93.2 | 1 (C1) |

This spectrum supports the structure of the compound as containing an intact glucose moiety, as would be expected for a phosphate sugar salt.

c. Phosphorus-31

A doublet peak was located at 7.85 and 7.95 ppm, respectively with a J value (measure of the degree of peak separation) of 15.9 Hz, characteristic of the phosphorus being in a P-O-Sn alignment within the molecule. This would be expected for the Sn being closely associated (bound) to the phosphate group.

d. Tin-119

A singlet peak was located at - 693.O ppm, which is characteristic of a stannous phosphate species.

4. Microanalysis data:

This batch was analysed for carbon and hydrogen content using a Carlo Erba Strumentazione E.A.1106 CHN Analyser:

| Atom | (Weight %) | |
|---|---|---|
| | Expected | Actual |
| carbon | 16.7 | 16.9 |
| hydrogen | 3.44 | 3.28 |

Therefore, it fits that this molecule is a trihydrate species.

5. Mossbauer spectra (for $Sn^{2+}$ content)

For this compound, only a tight doublet peak at 3.14 $mmsec^{-1}$ was observed, indicating that virtually all of the tin present was in the form of $Sn^{2+}$. Using an iodine titration method, it was found that this compound contained one mole of $Sn^{2+}$ per mole of glucose 6-phosphate trihydrate.

**EXAMPLE 2**

In the same manner as in Example 1, stannous glucose-1-phosphate (Sn G-1-P) was prepared from disodiumglucose-1-phosphate and stannous chloride.

**EXAMPLE 3**

In a similar manner as in Example 1, the stannous salts of glucose-1,6-diphosphate, fructose-6-phosphate, fructose-1,6-diphosphate and fructose-2,6-diphosphate are prepared.

5

## EXAMPLE 4

Substituting copper or zinc for the stannous ion in Example 1 gave the corresponding copper and zinc salts. Analysis by atomic absorption and infrared spectrometry showed these salts to be simple copper and zinc glucose-6-phosphate salts.

## EXAMPLE 5

Replacing the glucose- or fructose-phosphates of Example 1-4 by a mixture of mono-, di- and pyrophosphorylated saccharides gave similar results.

## EXAMPLE 6

Monostannous glucose-6-phosphate and monostannous glucose-1-phosphate were assessed in the in vitro pH-Stat. assay as to their inhibition of acid production by S.mutans. For comparison purpose stannous pyrophosphate was also assessed in the pH-Stat. assay. The method followed is the method described in Caries Research 25 (1991), pages 431-437.

The following results were obtained (at pH = 7):

Concentration of $Sn^{2+}$ in mmole to reach 100 % acid inhibition

| | |
|---|---|
| $Sn_2P_2O_7$ | 1.02 mmole |
| Sn G-1-P | 0.2 mmole |
| Sn G-6-P | 0.25 mmole. |

## EXAMPLE 7

The same materials as in Example 6 were tested in an in vitro anti-plaque test involving saliva reinoculation.

In this test a small amount of plaque bacteria was applied onto enamel discs (to mimic a tooth surface). The plaques were grown on these discs in a warm (37°C), humid chamber overnight (20 hrs), dripping a nutrient broth onto the plaque (mimics growth at an air-water-solid interface, continually bathed in nutrients from saliva), and following this overnight growth, the active stannous salt was administered to the plaque, and allowed to sit for 20 minutes. A small amount of saliva was then applied to the plaque to mimic the washing effect of saliva in the mouth, and the number of plaque bacteria on discs treated with active compounds was determined, and compared to the number on untreated plaques (to get a % killed by the active salt).

The results obtained using this method were as follows:

### Relative Anti-Plaque Activity of Stannous Salts
### (% bacteria killed vs. control growth)

| (ppm) $Sn^{II}$ conc. | $Sn^{II}$ pyrophosphate | $Sn^{II}$ glucose 6-$PO_4$ | $Sn^{II}$ glucose 1-$PO_4$ |
|---|---|---|---|
| 1188 | * | | 32 |
| 2406 | | 22 | |
| 3594 | | 75 | 96 |
| 6000 | 76 | 88 | 98 |

```
*     a blank space means that either this concentration was
      not tested (glucose phosphates), or the dose response
      was unsuitable (pyrophosphate).
```

### EXAMPLE 8

Similar results are obtained in Example 6 and 7 if the compounds according to Example 4 are used.

### Claims

1. Sn, Zn and Cu-salts of polyhydroxy compounds having at least 4 carbon atoms and having at least one acid, ester-linked salt-forming substituent, excluding Zn-hexosephosphates and stannous glucose-1-phosphate.

2. The salts according to claim 1, wherein the polyhydroxy compound is a saccharide.

3. The salts according to claim 1 or 2, wherein the polyhydroxy compound is substituted by at least one ester-linked phosphoric acid substituent.

4. The salts according to claim 2 or 3, wherein the polyhydroxy compound is selected from the group consisting of glucose, fructose, arabinose, mannose and galactose.

5. The salts according to claim 1 being represented by the Sn or Cu salts of glucose-1-phosphate, glucose-6-phosphate, glucose-1,6-diphosphate, fructose-1-phosphate, fructose-6-phosphate, fructose-1,6-diphosphate.

6. Sn, Cu and Zn-salts of polyhydroxy compounds having at least 4 carbon atoms and having at least one acid, ester-linked salt-forming substituent for the treatment of oral disorders.

7. The salts of claim 6 for the prevention or reduction of dental plaque, bath breath, dental caries and gingivitis.

8. Oral care compositions comprising an effective amount of a salt according to claim 1.

9. The salts of claim 6 for use as anti-microbial agents in personal care compositions, other than oral care compositions.

10. Personal care compositions, other than oral care compositions, comprising an effective amount of a salt according to claim 1.

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | PATENT ABSTRACTS OF JAPAN vol. 015, no. 123 (C-0816)26 March 1991 & JP-A-03 007 214 (KAO CORP) 14 January 1991 * abstract * | 1-8 | C07H11/04 A61K7/16 A61K7/06 A61K7/32 A61K7/48 |
| X | PATENT ABSTRACTS OF JAPAN vol. 017, no. 391 (C-1087)22 July 1993 & JP-A-05 070 358 (KAO CORP) 23 March 1993 * abstract * | 1-8 | |
| X | CHEMICAL ABSTRACTS, vol. 115, no. 10, 9 September 1991, Columbus, Ohio, US; abstract no. 99027s, S. TSUJITA ET AL 'Stable dentifrices containing zinc salts and sugar phosphates.' page 429 ;column 2 ; * abstract * & JP-A-0 307 214 (KAO CORPORATION) | 1-8 | |
| X | CHEMICAL ABSTRACTS, vol. 119, no. 8, 23 August 1993, Columbus, Ohio, US; abstract no. 80289q, S. KAYANE ET AL 'Dental sealants containing zinc hydroxide and zinc oxide colloids and zinc salts.' page 523 ;column 1 ; * abstract * & JP-A-0 570 358 (KAO CORPORATION) | 1-6 | TECHNICAL FIELDS SEARCHED (Int.Cl.6) C07H A61K |
| X A | EP-A-0 194 710 (LABORATORIOS VINAS) * claims; examples * | 1 2-4 | |
| A | GB-A-2 216 005 (THE PROCTER & GAMBLE COMPANY) | 1,6 | |

The present search report has been drawn up for all claims

| Place of search | Date of completion of the search | Examiner |
|---|---|---|
| THE HAGUE | 26 May 1994 | Moreno, C |

EPO FORM 1503 03.82 (P04C01)